# EUROPEAN PATENT APPLICATION

(11) **EP 4 775 137 A1**
(43) Date of publication of application: **15.07.2026**
(21) Application number: 24862384.5
(22) Date of filing: 14.05.2024
(51) Int. Cl.: A61B 5/02

(54) **PULSE WAVE MEASUREMENT DEVICE**

(30) Priority: 07.09.2023 US 202363581104 P
(71) Applicant: Minebea Mitsumi Inc., Kitasaku-gun, Nagano 3890293 (JP)
(72) Inventor: NAGAI, Takuya, Kitasaku-gun, Nagano 389-0293 (JP)
(74) Representative: Winter, Brandl - Partnerschaft mbB
(86) International application number: PCT/JP2024/017848
(87) International publication number: WO 2025/052727

(57) **Abstract**

A pulse wave measuring device that allows a pulse wave sensor to be brought to an appropriate position relative to the user's radial artery, regardless of the size of the user's wrist, is provided. A pulse wave measuring device (100) includes: a first arm (10) and a second arm (20) configured to swing relatively about a first shaft extending in a first direction, the first direction paralleling a longitudinal direction of the radial artery (110); a pulse wave sensor (31) provided at a distal end of the first arm (10); and a moving part (40) connected to the second arm (20) and configured to move toward or away from the pulse wave sensor (31).

## Description

### TECHNICAL FIELD

The present disclosure relates to a pulse wave measuring device.

### BACKGROUND ART

For example, there is an electrode holding tool for holding electrodes that are for use on a living body and that are brought into contact with target parts of the user's body when in use (see, for example, patent document 1). This electrode holding tool includes: a first arm and a second arm that open and close by rotating relatively about a first rotation fulcrum and bring the electrodes into contact with target parts of the user's body; and a biasing member that exerts a biasing force in a direction in which the first arm and the second arm close.

### CITATION LIST

### PATENT DOCUMENTS

Patent Document 1: Unexamined Japanese Patent Application Publication No. 2020-163012

### SUMMARY OF THE INVENTION

### PROBLEM TO BE SOLVED BY THE INVENTION

For example, there is a pulse wave measuring device with a pulse wave sensor for detecting pulse waves that are produced when the user's heart pumps blood. A pulse wave measuring device of this kind is worn, for example, on the user's wrist. The pulse wave sensor needs to detect minute signals, so it is desirable to place it in an appropriate position relative to the user's radial artery for improved accuracy of measurement. Because wrist circumference varies from person to person, there is a demand for a pulse wave measuring device that allows a pulse wave sensor to be brought to an appropriate position regardless of the size of the user's wrist.

The present disclosure therefore aims to provide a pulse wave measuring device that, regardless of the size of the user's wrist, allows a pulse wave sensor to be brought to an appropriate position relative to the user's radial artery.

### MEANS FOR SOLVING THE PROBLEM

According to an embodiment of the present disclosure, a pulse wave measuring device includes: a first arm and a second arm configured to swing relatively about a first shaft extending in a first direction, the first direction paralleling a longitudinal direction of a radial artery; a pulse wave sensor provided at a distal end of the first arm; and a moving part connected to the second arm and configured to move toward or away from the pulse wave sensor.

### ADVANTAGEOUS EFFECTS OF THE INVENTION

According to the technique disclosed herein, it is possible to provide a pulse wave measuring device that, regardless of the size of the user's wrist, allows a pulse wave sensor to be brought to an appropriate position relative to the user's radial artery.

### BRIEF DESCRIPTION OF THE DRAWINGS

[FIG. 1] FIG. 1 is a side view illustrating a pulse wave measuring device according to an embodiment of the present disclosure;
[FIG. 2] FIG. 2 is a perspective view illustrating the pulse wave measuring device of the embodiment, showing a state in which a moving part is not erect;
[FIG. 3] FIG. 3 is a perspective view illustrating the pulse wave measuring device of the embodiment, showing a state in which the moving part is erect;
[FIG. 4] FIG. 4 is a side view illustrating a second arm and the moving part; and
[FIG. 5] FIG. 5 is a perspective view illustrating the pulse wave measuring device of the embodiment, seen from the outer side of the second arm.

### DESCRIPTION OF EMBODIMENTS

An embodiment of the present invention will be described below with reference to the accompanying drawings. Throughout the drawings, the same components will be assigned the same reference numerals and will not be explained twice.

### [Pulse wave measuring device 100 according to embodiment]

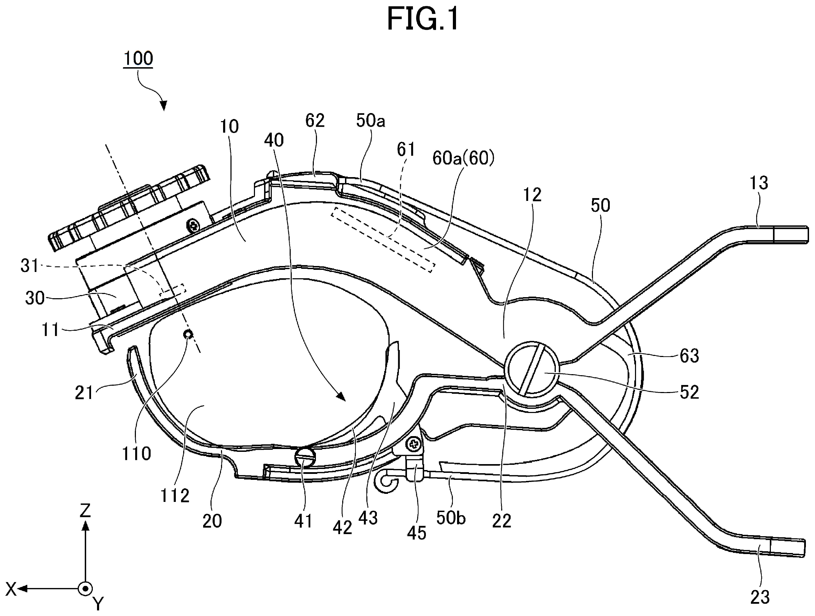

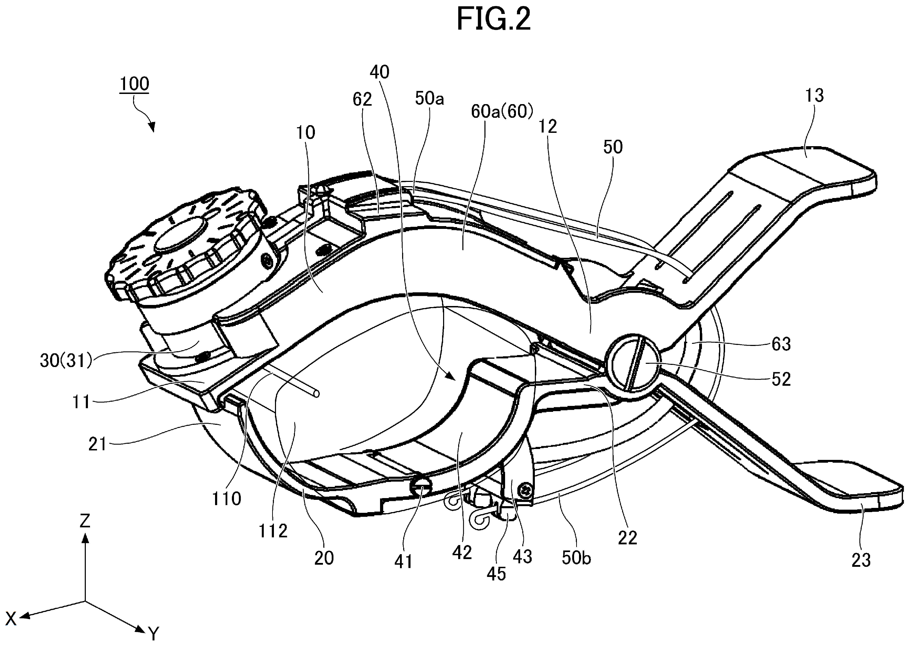

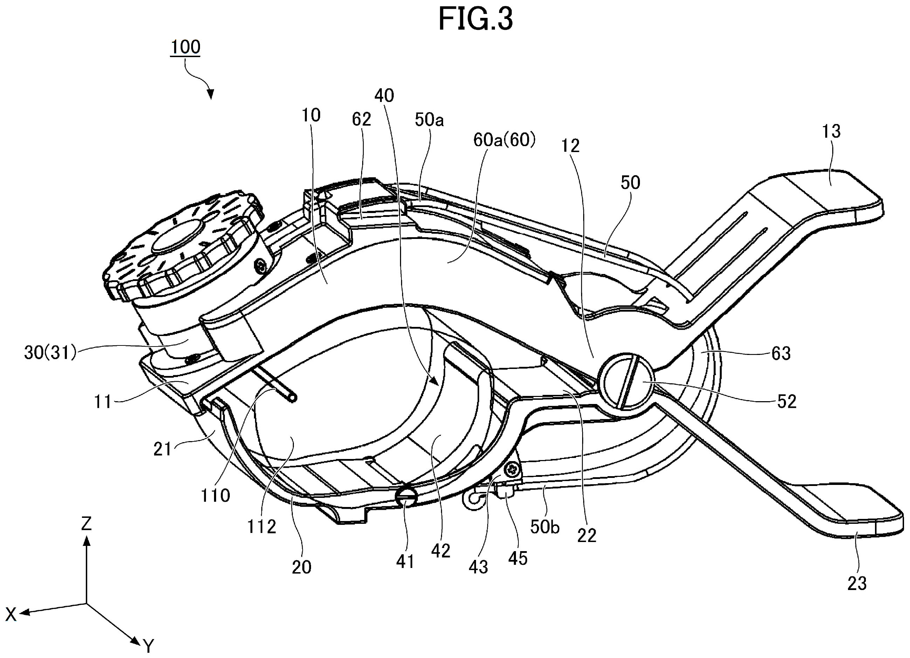

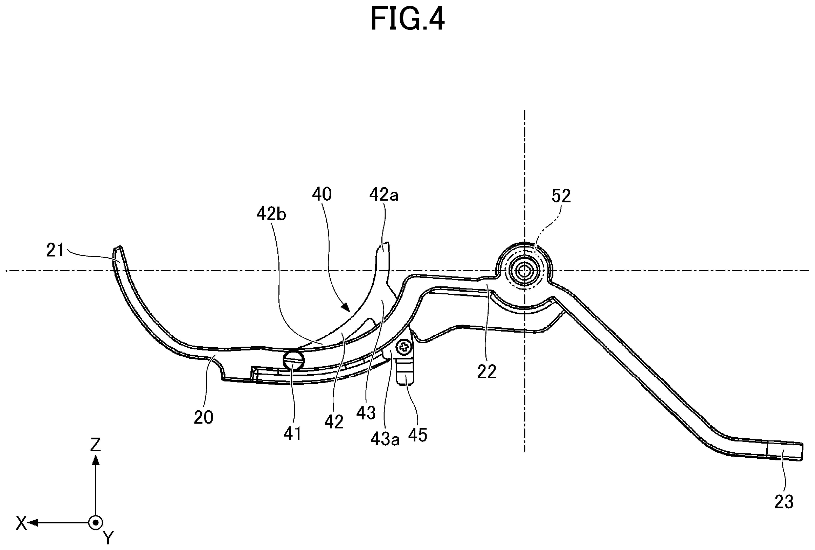

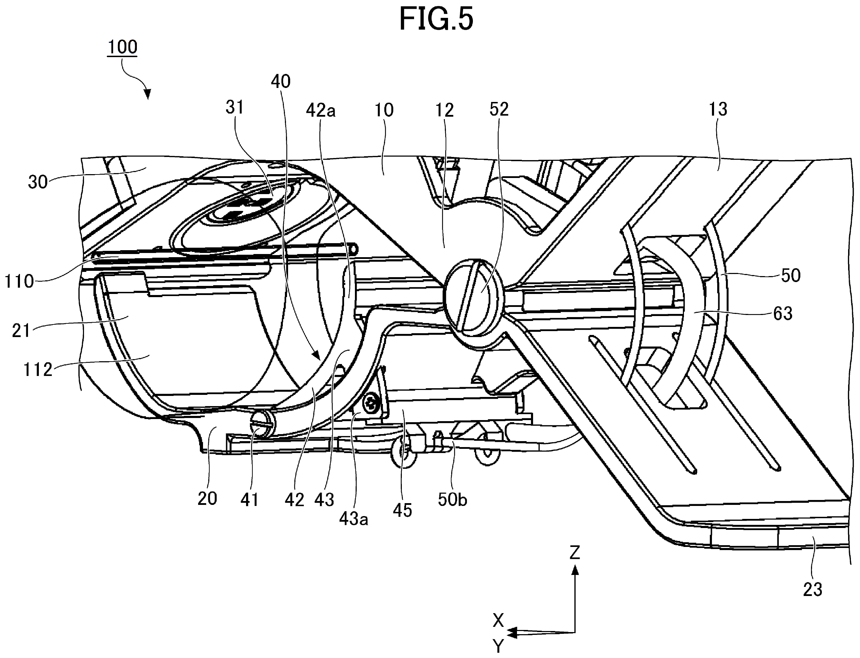

FIG. 1 is a side view illustrating a pulse wave measuring device 100 according to an embodiment of the present invention. FIG. 2 is a perspective view illustrating the pulse wave measuring device 100 of the embodiment, showing a state in which a moving part 40 is not erect. FIG. 3 is a perspective view illustrating the pulse wave measuring device 100 of the embodiment, showing a state in which the moving part 40 is erect. FIG. 4 is a side view illustrating a second arm 20 and the moving part 40. FIG. 5 is a perspective view illustrating the pulse wave measuring device 100 of the embodiment, seen from the outer side of the second arm 20. Note that each figure shows an X-axis direction, a Y-axis direction, and a Z-axis direction, which are mutually orthogonal to each other. The Y-axis direction parallels a first direction, which is the longitudinal direction of the user's radial artery.

In addition, in the accompanying drawings, an arrow may indicate a positive direction, and a direction opposite thereto may indicate a negative direction. The X-axis direction refers to both the positive X-axis direction and the negative X-axis direction. The positive X-axis direction and the negative X-axis direction are mutually opposite directions. The Y-axis direction refers to both the positive Y-axis direction and the negative Y-axis direction. The positive Y-axis direction and the negative Y-axis direction are mutually opposite directions. The Z-axis direction refers to both the positive Z-axis direction and the negative Z-axis direction. The positive Z-axis direction and the negative Z-axis direction are mutually opposite directions.

The pulse wave measuring device 100 shown in FIG. 1 to FIG. 3 is a wearable device that a user can wear on him/herself. The pulse wave measuring device 100 is worn, for example, on the user's wrist 112 such that a pulse wave sensor 31, which includes a strain element, is brought into contact with the skin above the user's radial artery 110. A pulse wave is a waveform that represents a change in blood vessel volume that occurs when the user's heart pumps blood. The pulse wave measuring device 100 enables monitoring of changes in blood vessel volume.

The pulse wave measuring device 100 includes a first arm 10, a second arm 20, a pulse wave sensor 31, and a moving part 40. The pulse wave measuring device 100 may be a clip-like pulse wave measuring device 100 that sandwiches the user's wrist 112 between the first arm 10 and the second arm 20. The pulse wave measuring device 100 has a swing shaft 52. The first arm 10 and the second arm 20 can "swing" about the swing shaft 52 (that is, the first arm 10 and the second arm 20 can make up-and-down or "seesaw" movements relative to the swing shaft 52 as a fulcrum).

### [Pulse wave sensor mounting part 30]

The pulse wave measuring device 100 includes a pulse wave sensor mounting part 30 in which the pulse wave sensor 31 is placed. The pulse wave sensor 31 detects the user's pulse waves. The pulse wave sensor mounting part 30 is provided at a distal end 11 of the first arm 10. The pulse wave sensor mounting part 30 may include an angle adjusting mechanism that allows the angle of the pulse wave sensor 31 to be adjusted.

### [Swing shaft 52]

The swing shaft 52 extends in the Y-axis direction. The Y-axis direction is the direction in which the radial artery 110 extends. The first arm 10 and the second arm 20 can swing about the swing shaft 52. The swing shaft 52 is an example of a first shaft. The first arm 10 and the second arm 20 can swing relatively about the swing shaft 52. Saying that the first arm 10 and the second arm 20 can swing "relatively" means that at least one of the first arm 10 and the second arm 20 can swing about the swing shaft 52.

### [First arm 10]

In the circumference of the user's wrist 112, the first arm 10 is positioned to face part of the circumference that is located closer to the palm of the hand. Seen from the Y-axis direction, the first arm 10 is bent to draw an upward curve in the positive Z-axis direction. The first arm 10 is positioned such that its longitudinal direction and the circumferential direction of the user's wrist 112 parallel each other. The first arm 10 has a predetermined width in the Y-axis direction. The first arm 10 may include a flat portion having a predetermined thickness. The first arm 10 may also have a rib that protrudes in the thickness direction. The first arm 10 may have multiple ribs that are positioned at intervals in the Y-axis direction. The ribs may be a pair of side plates 60a, which will be described later.

The first arm 10 has a distal end 11 and a proximal end 12. In the longitudinal direction of the first arm 10, the proximal end 12 is the end located closer to the swing shaft 52, and, conversely, the distal end 11 is the end located farther from the swing shaft 52.

When viewed in the Y-axis direction, the first arm 10 may include a portion that extends linearly and a portion that is curved. The first arm 10 may include: a linearly-extending first portion that extends away from the swing shaft 52; a second portion that curves from the first portion; and a third portion that extends linearly from the second portion. The first arm 10 may be formed to draw a curve as a whole, or may be formed such that multiple straight lines are joined one after another at predetermined angles. Looking at the thickness direction of the first arm 10, the side located closer to the user's wrist 112 is the "inner side", and the side located farther from the user's wrist 112 is the "outer side".

### [Second arm 20]

Along the circumference of the user's wrist 112, the second arm 20 is positioned to face a portion of the circumference that is located closer to the back of the hand. The second arm 20 is positioned opposite the first arm 10 so that the user's wrist 112 is sandwiched between the two arms. Seen from the Y-axis direction, the second arm 20 is bent to draw an upward curve in the negative Z-axis direction. The second arm 20 is positioned such that its longitudinal direction and the circumferential direction of the user's wrist 112 parallel each other. The second arm 20 has a predetermined width in the Y-axis direction. The second arm 20 may include a flat portion having a predetermined thickness. The second arm 20 may also have a rib that protrudes in the thickness direction. The second arm 20 may have multiple ribs that are positioned at intervals in the Y-axis direction.

The second arm 20 has a distal end 21 and a proximal end 22. In the longitudinal direction of the second arm 20, the proximal end 22 is the end located closer to the swing shaft 52, and, conversely, the distal end 21 is the end located farther from the swing shaft 52.

As shown in FIG. 4, viewed from the Y-axis direction, the second arm 20 may include a portion that extends linearly in the X-axis direction and a portion that is curved. The second arm 20 may include: a linearly-extending first portion that extends away from the swing shaft 52; a second portion that is bent from the first portion to part from the first arm 10 and draw a curve along the circumference of the user's wrist 112; and a third portion that continues from the second portion and extends linearly; and a fourth portion that continues from the third portion and draws a curve along the circumference of the user's wrist 112. The second arm 20 may be formed such that the second arm 20 is curved as a whole, or the second arm 20 may be formed by connecting multiple straight lines one after another at predetermined angles.

### [First handle part 13 and second handle part 23]

As shown in FIG. 1 to FIG. 3, the pulse wave measuring device 100 includes a first handle part 13 and a second handle part 23. The first handle part 13 is connected to the proximal end 12 of the first arm 10 and extends opposite the first arm 10. The first handle part 13 is, for example, formed integrally with the first arm 10 and swings together with the first arm 10. The first handle part 13 can swing about the swing shaft 52. The first arm 10 and the first handle part 13 are made of, for example, resin. The material of the pulse wave measuring device 100 is not limited.

The second handle part 23 is connected to the proximal end 22 of the second arm 20 and extends opposite the second arm 20. The second handle part 23 is, for example, formed integrally with the second arm 20 and swings together with the second arm 20. The second handle part 23 can swing about the swing shaft 52. The second arm 20 and second handle part 23 are made of, for example, resin.

### [Open state of pulse wave measuring device 100]

The pulse wave measuring device 100 can be operated to bring the first handle part 13 and the second handle part 23 closer to each other. When the first handle part 13 and the second handle part 23 swing in a way that brings them closer to each other (that is, when the first handle part 13 and the second handle part 23 are pinched together), the distal end 11 of the first arm 10 and the distal end 21 of the second arm 20 move apart from each other (that is, open). When the distal end 11 of the first arm 10 and the distal end 21 of the second arm 20 are apart from each other in this manner, the pulse wave measuring device 100 is in the "open state". When the user wears the pulse wave measuring device 100 on his/her wrist 112, the pulse wave measuring device 100 is placed in the open state, so that the first arm 10 and the second arm 20 can sandwich the user's wrist 112.

### [Closed state of pulse wave measuring device 100]

The pulse wave measuring device 100 can also be operated such that the first handle part 13 and the second handle part 23 move apart from each other (that is, open). When the first handle part 13 and the second handle part 23 swing in a way that parts them from each other, the distal end 11 of the first arm 10 and the distal end 21 of the second arm 20 move closer to each other. When the distal end 11 of the first arm 10 and the distal end 21 of the second arm 20 are thus brought close to each other, the pulse wave measuring device 100 is in the "closed state". When the pulse wave measuring device 100 is worn on the user's wrist 112, the pulse wave measuring device 100 is in the closed state. The pulse wave measuring device 100 is in the closed state while measuring the user's pulse waves.

### [Biasing member 50]

The pulse wave measuring device 100 has a biasing member 50. The biasing member 50 biases at least one of the first arm 10 and the second arm 20 in a direction in which the arms come nearer to each other. The biasing member 50 may be, for example, a wire spring. The biasing member 50 may be a leaf spring, a torsion spring, or any other spring. The biasing member 50 is formed, for example, from metal. The biasing member 50 has a predetermined length. One longitudinal end of the biasing member 50 is connected to the first arm 10, and the other longitudinal end is connected to the second arm 20. The biasing member 50 may be connected to the first arm 10 or the second arm 20 via an additional member.

### [Moving part 40]

As shown in FIG. 1 to FIG. 5, the pulse wave measuring device 100 has a moving part 40 that is connected to the second arm 20 and that can move or be moved relative to the second arm 20. Seen from the Y-axis direction, the moving part 40 can move toward or away from the pulse wave sensor 31. FIG. 1 and FIG. 3 to FIG. 5 all show a state in which the moving part 40 is positioned close to the pulse wave sensor 31. FIG. 2 shows a state in which the moving part 40 is positioned far from the pulse wave sensor 31.

### [First state, second state, and third state of moving part 40]

The state in which the moving part 40 is positioned closest to the pulse wave sensor 31 and a receiving part 45 (which will be described later) of the moving part 40 and in contact with the outer side/surface of the second arm 20 will be hereinafter referred to as the "first state". Also, as shown in FIG. 2, the state in which the moving part 40 is positioned farthest from the pulse wave sensor 31 and a push plate 42 (which will be described later) of the moving part 40 is housed in the second arm 20 will be hereinafter referred to as the "third state". Furthermore, referring again to FIG. 1 and FIG. 3 to FIG. 5, the state in which the moving part 40 is positioned midway between the first state and the third state will be hereinafter referred to as the "second state". In this way, the moving part 40 can move (swing) between the first state, the second state, and the third state.

### [Second swing shaft 41]

The moving part 40 has a second swing shaft 41. The second swing shaft 41 extends in the Y-axis direction. The moving part 40 can swing about the second swing shaft 41. The second swing shaft 41 is an example of a second shaft. The second swing shaft 41 is located on the opposite side of the pulse wave sensor 31 with respect to the radial artery 110. For example, the distance from the user's radial artery 110 to the second swing shaft 41 is greater than the distance from the user's radial artery 110 to the pulse wave sensor 31. The moving part 40 is held by the second arm 20. The moving part 40 is located in a middle part of the second arm 20 in the longitudinal direction. The middle part of the second arm 20 may be located midway between the distal end 21 and the proximal end 22.

### [Push plate 42]

The moving part 40 has a push plate 42 and a protruding part 43. The push plate 42 extends in a direction along the circumference of the user's wrist 112. The push plate 42 can contact part of the circumferential surface of the user's wrist 112. Seen from the Y-axis direction, the push plate 42 is curved to parallel part of the circumference of the user's wrist 112. The push plate 42 has a distal end 42a and a proximal end 42b in the longitudinal direction of the push plate 42. In the thickness direction of the second arm 20, the side/surface of the push plate 42 located closer to the user's wrist 112 will be referred to as the "inner side/surface" of the second arm 20, and the side/surface located farther from the user's wrist 112 will be hereinafter referred to as the "outer side/surface". The inner side/surface of the push plate 42 can contact the circumferential surface of the user's wrist 112.

The end of the push plate 42 that is located closer to the second swing shaft 41 is the proximal end 42b of the push plate 42, and the end that is located farther from the second swing shaft 41 is the distal end 42a. The proximal end 42b is connected to the second swing shaft 41. An opening in which the second swing shaft 41 can be inserted is formed at the proximal end 42b. The push plate 42 is supported by the second arm 20 such that the push plate 42 can swing about the second swing shaft 41.

As shown in FIG. 2 and FIG. 3, the push plate 42 may be narrower in width than the second arm 20. The widths of the second arm 20 and the push plate 42 are dimensions in the Y-axis direction. In the third state shown in FIG. 2, the push plate 42 is housed in the second arm 20. A recess (terraced part), in which the push plate 42 can be housed, may be formed in the second arm 20.

### [Protruding part 43]

As shown in FIG. 1 to FIG. 5, the protruding part 43 protrudes from the push plate 42, in the opposite direction from the user's wrist 112. An opening in which the protruding part 43 can be inserted may be formed in the second arm 20. The protruding part 43 penetrates the second arm 20 and protrudes outward from the second arm 20. Seen from the Y-axis direction, the protruding part 43 is formed to intersect with the second arm 20. The push plate 42 and the protruding part 43 may be formed, for example, in the shape of an inverted Y seen from the Y-axis direction.

The end of the protruding part 43 located closer to the push plate 42 is the proximal end of the protruding part 43, and the end located farther from the push plate 42 is the distal end 43a. The distal end 43a is located opposite the push plate 42. The moving part 40 may have, for example, a pair of protruding parts 43. The pair of protruding parts 43 are formed apart from each other in the Y-axis direction.

### [Receiving part 45]

The moving part 40 has a receiving part 45. The receiving part 45 extends in the Y-axis direction and connects the respective distal ends 43a of the pair of protruding parts 43 together. The receiving part 45 may be, for example, a member shaped like a stick. Both longitudinal ends of the receiving part 45 are fixed to the distal ends 43a of the pair of protruding parts 43. The receiving part 45 is, for example, screwed to the distal ends 43a of the protruding parts 43.

The other end 50b of the biasing member 50 is connected to the receiving part 45. The end 50b of the biasing member 50 is connected to the second arm 20 via the receiving part 45, the protruding parts 43, the push plate 42, and the second swing shaft 41. In other words, the biasing member 50 applies a biasing force to the receiving part 45, which is then transmitted from the receiving part 45 to the second arm 20 via the protruding parts 43, the push plate 42, and the second swing shaft 41. The moving part 40 can swing about the second swing shaft 41 but is constantly biased by the biasing member 50 toward the pulse wave sensor 31. Being thus biased by the biasing member 50, the moving part 40 can change its position. The moving part 40 can switch between the first state, the second state, and the third state.

In the third state, the receiving part 45 is positioned farthest from the second arm 20. In the first state, the receiving part 45 is in contact with the outer side/surface of the second arm 20. When the receiving part 45 is in contact with the outer side/surface of the second arm 20, the range of movement of the moving part 40 is limited.

In the third state, the push plate 42 is in contact with the inner side/surface of the second arm 20. When the push plate 42 is in contact with the inner side/surface of the second arm 20, movement of the moving part 40 away from the pulse wave sensor 31 is restricted.

### [Wearing pulse wave measuring device 100 (for thin wrist)]

Next, the procedures to follow when wearing the pulse wave measuring device 100 on a thin wrist will be described with reference to FIG. 1, FIG. 3, and FIG. 4. First, assuming that the pulse wave measuring device 100 is not worn on the user's wrist 112 and the first arm 10 and the second arm 20 are closed, the moving part 40 is in the third state. In this state, the push plate 42 is constantly biased toward the pulse wave sensor 31 by the biasing member 50, so that the receiving part 45 contacts the outer side/surface of the second arm 20. Subsequently, by operating the first handle part 13 and the second handle part 23 and placing the first arm 10 and the second arm 20 in the open state, the user is now able to wear the pulse wave measuring device 100 on his/her wrist 112. To wear the pulse wave measuring device 100, part of the circumferential surface of the user's wrist 112 near the farthest point from the user's radial artery 110 is brought into contact with the inner side/surface of the push plate 42, and the push plate 42 is pushed further against the biasing force of the biasing member 50, thus causing the push plate 42 to swing away from the pulse wave sensor 31.

Next, part of the circumferential surface of the user's wrist 112 on the side nearer to the user's radial artery 110 and the back of his/her hand may be slid into contact with the distal end 21 of the second arm 20. The pulse wave measuring device 100 can be worn thus. As mentioned earlier, the push plate 42 is constantly biased by the biasing member 50 toward the pulse wave sensor 31, so the push plate 42 is pressed against the user's wrist 112 such that there is no longer a gap between the user's wrist 112 and the distal end 21 of the second arm 20. As a result of this, even if the user's wrist is thin, the push plate 42 can move or be moved in a way that accommodates the thinness of the user's wrist, so that, regardless of the circumference of the user's wrist 112, the pulse wave sensor 31 can be brought to an appropriate position relative to the user's radial artery 110. When the pulse wave measuring device 100 is worn on the user's wrist 112, the receiving part 45 is separate from the outer side/surface of the second arm 20, and the push plate 42 is not in contact with the inner side/surface of the second arm 20, the pulse wave measuring device 100 is in the second state.

The second state, for example, places a part of the second arm 20 located nearer to the distal end 21 and a part of the push plate 42 in contact with the circumferential surface of the user's wrist 112. The pulse wave measuring device 100 is positioned appropriately relative to the user's wrist 112, and the pulse wave sensor 31 is positioned near the radial artery 110. In this state, the second arm 20 and the push plate 42 come into contact with the circumferential surface of the user's wrist 112, resulting in an increased contact area. Consequently, the frictional resistance between the user's wrist 112 and the second arm 20 and between the user's wrist 112 and the push plate 42 increases. This provides an advantage of preventing or substantially preventing the pulse wave measuring device 100 from rotating around the user's wrist 112 even when the center of gravity of the pulse wave measuring device 100 is offset from the axial center of the user's wrist 112.

### [Wearing pulse wave measuring device 100 (for thick wrist)]

Next, how to wear the pulse wave measuring device 100 on a thick wrist 112 will be described with reference to FIG. 2. The procedures to follow to wear the pulse wave measuring device 100 are substantially the same as described above, regardless of the size of the user's wrist 112. However, if the user's wrist 112 is as thick as shown in FIG. 2, the moving part 40 may not stay in the second state and may enter the third state. In other words, depending on the thickness of the user's wrist 112, the push plate 42 of the moving part 40 may be pushed away from the user's radial artery 110, against the biasing force from the biasing member 50, and come into contact with the inner side/surface of the second arm 20. The push plate 42 contacts the circumferential surface of the user's wrist 112 and moves nearer to the second arm 20. To be more specific, the distal end 42a of the push plate 42 moves closer to the proximal end 22 of the second arm 20.

### [Movement of moving part 40 toward pulse wave sensor 31]

As described above, the moving part 40 can transition from the first state to the second state, and from the second state to the third state. In the first state of the pulse wave measuring device 100, which is the initial state, the push plate 42 is positioned closest to the pulse wave sensor 31. For example, when a user with a thick wrist wears the pulse wave measuring device 100 on his/her wrist and then removes it, the push plate 42 moves closer to the pulse wave sensor 31. That is, the push plate 42 moves nearer to the user's radial artery 110. The push plate 42, being biased by the biasing member 50, is pushed away from the second arm 20, and pushed toward the circumferential surface of the user's wrist 112. This allows, when the pulse wave measuring device 100 is worn on the user's wrist 112, the push plate 42 to contact the circumferential surface of the user's wrist 112.

### [Movement of moving part 40 to part from pulse wave sensor 31]

For example, when the moving part 40 switches from the initial first state to the second or the third state, the push plate 42 of the moving part 40 moves away from the pulse wave sensor 31. By wearing the pulse wave measuring device 100 on his/her wrist 112, the user can move the push plate 42 away from the pulse wave sensor 31.

### [Substrate mounting part 60]

The first arm 10 has a substrate mounting part 60, on which a wiring substrate 61 that is electrically connected to the pulse wave sensor 31 is mounted. The first arm 10 includes a pair of side plates 60a that are positioned apart from each other in the width direction of the first arm 10. The wiring substrate 61 is mounted between the pair of side plates 60a. The substrate mounting part 60 may also be a housing that houses the wiring substrate 61 inside. The wiring substrate 61 is covered by a cover 62. The cover 62 is fixed to the pair of side plates 60a.

For example, parts/elements that function in pulse wave detection may be provided on the wiring substrate 61. Examples of such parts/elements include: an amplifier circuit for amplifying an output of a strain gauge; an AD converter for converting an output of the amplifier circuit into a digital signal; a signal processing semiconductor for processing the digital signal; and a wireless communication semiconductor for sending the outcome of signal processing to outside. A cable 63 may be connected to the wiring substrate 61. The cable 63 can be used, for example, to ensure a supply of power, to send and receive electrical signal outputs and inputs to and from outside circuits, and so forth. Instead of using the cable 63, a battery may be installed to access an adequate supply of power.

In addition, one end 50a of the biasing member 50 may be fixed to the cover 62. The biasing member 50 is connected to the first arm 10 via the cover 62.

### [Functions and effects of pulse wave measuring device 100 of embodiment]

The pulse wave measuring device 100 of the embodiment incudes: a first arm 10 and a second arm 20 that can swing relatively about a swing shaft (first shaft) 52 extending in the Y-axis direction (first direction), where the Y-axis direction parallels the longitudinal direction of the user's radial artery 110; a pulse wave sensor 31 that is provided at the distal end 11 of the first arm 10; and a moving part 40 that is connected to the second arm 20 and that can move toward or away from the pulse wave sensor 31.

With this pulse wave measuring device 100, the movement of the moving part 40 allows the pulse wave sensor 31 to be positioned appropriately relative to the user's radial artery 110, regardless of the size of the user's wrist 112. Moving the moving part 40 toward or away from the pulse wave sensor 31 allows the second arm 20 and the moving part 40 to fit the circumferential surface of the user's wrist 112 well regardless of the size of the user's wrist 112. Although, in previous cases, the position of the pulse wave sensor relative to the user's wrist 112 was adjusted by inserting a spacer or other member between the user's wrist 112 and the second arm 20, this pulse wave measuring device 100 can place the pulse wave sensor 31 in an appropriate position without an intervening spacer.

In the pulse wave measuring device 100, the moving part 40 may be provided on the second arm 20 and swing about a second swing shaft (second shaft) 41 that extends in the Y-axis direction. The pulse wave measuring device 100 structured thus allows the moving part 40 to swing about the second swing shaft 41, allowing the moving part 40 to fit the circumferential surface of the user's wrist 112 with greater ease.

Additionally, in the pulse wave measuring device 100, the second swing shaft 41 may be positioned opposite the pulse wave sensor 31 with respect to the radial artery 110. The pulse wave measuring device 100 structured thus allows the moving part 40 to move toward the user's wrist 112, from the side opposite the pulse wave sensor 31. With this pulse wave measuring device 100, the moving part 40 can be positioned such that the user's wrist 112 is sandwiched between the pulse wave sensor 31 and the moving part 40.

Furthermore, in the pulse wave measuring device 100, the moving part 40 may include: a push plate 42 that is connected to the second swing shaft 41, extends in a direction along the circumference of the user's wrist 112, and contacts part of the circumference of the user's wrist 112; and a protruding part 43 that is connected to the push plate 42 and extends from the push plate 42, in a direction that is opposite the user's wrist 112 and intersects with the second arm 20.

Structured thus, this pulse wave measuring device 100 can position the push plate 42 to parallel the circumferential surface (part of the circumference) of the user's wrist 112, thereby allowing the push plate 42 to fit the user's wrist 112 well. Furthermore, because the protruding part 43 protrudes in a direction that intersects with the second arm 20, the push plate 42 positioned inside the second arm 20 can be moved by operating the protruding part 43 that protrudes from the second arm 20. The push plate 42 can be driven from outside the second arm 20, so that the push plate 42 fits the user's wrist 112 well.

The pulse wave measuring device 100 may also include a biasing member 50 that biases at least one of the first arm 10 or the second arm 20 in a direction to bring the first arm 10 and the second arm 20 closer to each other. In the pulse wave measuring device 100 structured thus, the biasing member 50 can bias at least one of the first arm 10 or the second arm 20, bringing the first arm 10 and the second arm 20 closer to each other. The first arm 10 and the second arm 20, biased by the biasing member 50, can sandwich the user's wrist 112 together. Consequently, the user can wear the pulse wave measuring device 100 on his/her wrist 112 and measure pulse waves all by him/herself.

Furthermore, in the pulse wave measuring device 100, the biasing member 50 may be connected to the distal end 43a of the protruding part 43 located opposite the push plate 42. Structured thus, the pulse wave measuring device 100 can bias the push plate 42 by connecting together the biasing member 50 and the distal end 43a of the protruding part 43 that protrudes from the push plate 42. Furthermore, in the pulse wave measuring device 100, the second arm 20 and the biasing member 50 can be connected together via the protruding part 43 and the push plate 42. This allows the moving part 40 and the second arm 20 to move in coordinated motion. The pulse wave measuring device 100 makes it unnecessary to provide a biasing member for biasing the moving part 40 and a biasing member for biasing the second arm 20 separately. Consequently, the number of parts/elements in the pulse wave measuring device 100 can be reduced, which then leads to lower manufacturing costs.

In the pulse wave measuring device 100, the moving part 40 may include: a pair of protruding parts 43 positioned apart from each other in the Y-axis direction; and a receiving part 45, which extends in the Y-axis direction, which connects the respective distal ends 43a of the protruding parts 43 together, and which has the biasing member 50 connected thereto. Structured thus, this pulse wave measuring device 100 can connect the biasing member 50 to the second arm 20 via the receiving part 45, which connects the respective distal ends 43a of the protruding parts 43 together. Consequently, the force that acts on the push plate 42 can be distributed across the width of the push plate 42.

Furthermore, in the pulse wave measuring device 100, the receiving part 45 may limit the range of movement of the moving part 40 by contacting the second arm 20. Structured thus, this pulse wave measuring device 100 can limit the range of movement when the moving part 40 is moved closer to the pulse wave sensor 31. Consequently, for example, when the user's wrist 112 is thin, the range of movement of the moving part 40 can be limited so as not to constrict the user's wrist 112.

Furthermore, the pulse wave measuring device 100 may be shaped substantially in the form of a clip, and the first arm 10 and the second arm 20 may be positioned opposite each other across the user's wrist 112 and curved to parallel at least part of the user's wrist 112. Structured thus, this pulse wave measuring device 100 can form the first arm 10 and the second arm 20 such that the two arms trace a curve that parallels at least part of the circumference of the user's wrist 112. As a result of this, the pulse wave measuring device 100 can bring the first arm 10 and the second arm 20 into close contact with the user's wrist 112 with ease.

The pulse wave measuring device 100 may also include: a first handle part 13, which is connected to the proximal end 12 of the first arm 10, which extends opposite the first arm 10, and which can swing about a swing shaft (first shaft) 52; and a second handle part 23, which is connected to the proximal end 22 of the second arm 20, which extends opposite the second arm 20, and which can swing about the swing shaft 52. In the pulse wave measuring device 100 structured thus, the first arm 10 and the second arm 20 can be opened and closed by operating the first handle part 13 and the second handle part 23. The user can wear the pulse wave measuring device 100 on his/her wrist 112 with ease.

### [First alternative example]

Next, a first alternative example of the pulse wave measuring device 100 will be described. The pulse wave measuring device 100 of the above-described embodiment allows the moving part 40 to move by biasing the moving part 40 using the biasing member 50, the pulse wave measuring device 100 may also be structured without the biasing member 50. The pulse wave measuring device 100 may also be structured with a drive part for enabling the moving part 40 to move.

The first alternative example of the pulse wave measuring device 100 may include a second biasing member that biases the moving part 40. The second biasing member is different from the biasing member 50 described earlier. The second biasing member is positioned, for example, between the push plate 42 and the second arm 20, and biases the push plate 42 towards the pulse wave sensor 31. The second biasing member may be, for example, a compression coil spring, a leaf spring, a torsion spring, and so forth.

The second biasing member need not be a spring member and may be a different type of actuator. The actuator may be, for example, an electric motor. The pulse wave measuring device 100 may include, for example, an electric motor and a drive-force transmitting mechanism, and drive the moving part 40 by transmitting the drive force produced by the electric motor to the moving part 40 via the drive-force transmitting mechanism. In addition, in the pulse wave measuring device 100, an air bag may be provided between the push plate 42 and the second arm 20 and inflated using a pump, thereby driving the moving part 40.

### [Second alternative example]

Next, a second alternative example of the pulse wave measuring device 100 will be described. In the second alternative example of the pulse wave measuring device 100, a slip-stop part may be formed on the inner side/surface of the push plate 42. This pulse wave measuring device 100 increases the frictional resistance between the user's wrist 112 and the push plate 42, thus reliably preventing or substantially preventing the pulse wave measuring device 100 from rotating around the user's wrist 112. This pulse wave measuring device 100 can reduce the mispositioning of the pulse wave sensor 31.

The slip-stop part may be formed with, for example, recesses and projections. The pulse wave measuring device 100 may also include a push plate 42 made of a material that is not slippery. Furthermore, in this pulse wave measuring device 100, a slip-stop part may be formed on the inner side/surface of the first arm 10 and on the inner side/surface of the second arm 20.

### [Third alternative example]

Next, a third alternative example of the pulse wave measuring device 100 will be described. A weight part with an adjustable center of gravity may be formed in the second arm 20. The center of gravity may refer to, for example, a position where the pulse wave measuring device 100 worn on the user's wrist 112 can be balanced such that the pulse wave measuring device 100 is prevented, for example, from rotating around the user's wrist 112. For example, a weight part may be formed by making the thickness of the second arm 20 vary from part to part. Furthermore, a weight part may be formed at the distal end 21 of the second arm 20. For example, a weight part may be formed by forming ribs in varying positions and sizes in the second arm 20 to adjust the center of gravity of the pulse wave measuring device 100. According to this pulse wave measuring device 100, it is possible to prevent or substantially prevent the pulse wave measuring device 100 worn on the user's wrist 112 from, for example, rotating around the user's wrist 112, having the pulse wave sensor 31 mispositioned, etc.

### [Fourth alternative example]

Next, a fourth alternative example of the pulse wave measuring device 100 will be described. In the pulse wave measuring device 100, the width of the first arm 10 and the width of the second arm 20 may vary. For example, the second arm 20 may be narrower in width than the first arm 10. By making the positions of the ends of the second arm 20 and the positions of the ends of the first arm 10 in the width direction different, the user may be able to bend his/her wrist 112 with greater ease. That is, when the pulse wave measuring device 100 is worn on the user's wrist 112, the movement of the user's hand is not hindered.

Furthermore, in the pulse wave measuring device 100, the width of the second arm 20 and the width of the second handle part 23 may be different. The second arm 20 may be narrower in width than the second handle part 23.

### [Fifth alternative example]

Next, a fifth alternative example of the pulse wave measuring device 100 will be described. The pulse wave sensor mounting part 30 of the pulse wave measuring device 100 may include an angle adjusting mechanism that allows the angle of the pulse wave sensor 31 to be adjusted. By changing the angle of the pulse wave sensor 31, the orientation of the pulse wave sensor 31 relative to the user's radial artery 110 can be adjusted.

### [Common method for measuring pulse rate]

When measuring a pulse rate in a hospital or similar facilities, the subject's palm is placed facing up, with three fingers positioned to parallel the radial artery 110, and the pulse rate is measured in this state. During this measurement, the user's wrist 112 is bent from the palm toward the back of the hand.

With the pulse wave measuring device 100 of the fifth alternative example, the back of the user's hand is less likely to come into contact with the second arm 20, thereby reducing mispositioning of the pulse wave sensor 31. This pulse wave measuring device 100 enables accurate measurement of pulse waves.

### [Pulse wave sensor 31]

The pulse wave sensor 31 may include a housing, a strain element, and multiple strain gauges. The strain gauges may be, for example, four strain gauges. Output signals of the pulse wave sensor 31 are generated based on the strain gauges' outputs. The strain gauges are connected such that each strain gauge forms a leg of a bridge circuit, so that the bridge circuit can generate output signals (pulse wave signals) of the pulse wave sensor 31.

In the pulse wave sensor 31, the load part of the strain element is positioned near the user's radial artery 110. Load is applied to the load part in response to the user's pulse waves, causing elastic deformation of the beam part of the strain element. This changes the resistance value of the strain gauges' resistors. The pulse wave sensor 31 can detect pulse waves based on changes in the resistance values of the resistors of the strain gauges that occur following deformation of the beam part of the strain element. Pulse waves are detected as output signals representing periodic changes of voltage from the bridge circuit.

An example has been described above in which the pulse wave sensor 31 has four strain gauges and output signals are generated by connecting four full-bridge strain gauges together. However, a structure may also be employed in which the pulse wave sensor 31 has two half-bridge strain gauges and output signals are generated by connecting two half-bridge strain gauges together.

The embodiment and examples disclosed herein are illustrative in all respects and should not be construed as limiting in any way. Various omissions, substitutions, or changes may be made to the embodiment and examples disclosed herein without departing from the spirit and scope of the claims attached herewith.

Although examples have been described hereinabove in which the moving part 40 has a push plate 42 and protruding parts 43, the moving part 40 may be structured and configured differently. Furthermore, the moving part 40 need not be one that makes swinging movements and may be one that makes linear movements. The moving part 40 may also move linearly so as to be closer to the pulse wave sensor 31.

This application claims the benefit of and priority to U.S. Provisional Patent Application No. 63/581,104, filed September 7, 2023, which is incorporated herein by reference in its entirety.

### REFERENCE SIGNS LIST

- 100: pulse wave measuring device
- 10: first arm
- 13: first handle part
- 20: second arm
- 23: second handle part
- 30: pulse wave sensor mounting part
- 31: pulse wave sensor
- 40: moving part
- 41: second swing shaft (second shaft)
- 42: push plate
- 43: protruding part
- 43a: distal end (distal end of protruding part)
- 45: receiving part
- 50: biasing member
- 52: swing shaft (first shaft)
- 60: substrate mounting part
- 61: wiring substrate (substrate)
- 110: radial artery
- 112: wrist

## Claims

1. A pulse wave measuring device comprising:
a first arm and a second arm configured to swing relatively about a first shaft extending in a first direction, the first direction paralleling a longitudinal direction of a radial artery;
a pulse wave sensor provided at a distal end of the first arm; and
a moving part connected to the second arm and configured to move toward or away from the pulse wave sensor.

2. The pulse wave measuring device according to claim 1, wherein the moving part is configured to swing about a second shaft provided on the second arm and extending in the first direction.

3. The pulse wave measuring device according to claim 2, wherein the second shaft is positioned opposite the pulse wave sensor with respect to the radial artery.

4. The pulse wave measuring device according to claim 2 or 3, wherein the moving part includes:
a push plate connected to the second shaft, extending in a direction along a part of a circumference of a wrist, and configured to contact the part of the circumference of the wrist; and
a protruding part connected to the push plate and extending from the push plate, in a direction that is opposite the wrist and intersects with the second arm.

5. The pulse wave measuring device according to claim 4, wherein a slip-stop part is formed on a surface or a side of the push plate located nearer to the wrist.

6. The pulse wave measuring device according to any one of claims 1 to 5, further comprising a biasing member configured to bias at least one of the first arm or the second arm in a predetermined direction to bring the first arm and the second arm closer to each other.

7. The pulse wave measuring device according to claim 4 or 5, further comprising a biasing member configured to bias at least one of the first arm or the second arm in a predetermined direction to bring the first arm and the second arm closer to each other,
wherein the biasing member is connected to a distal end of the protruding part, the distal end being an end of the protruding part located opposite the push plate.

8. The pulse wave measuring device according to claim 7, wherein the moving part includes:
a pair of protruding parts positioned apart from each other in the first direction; and
a receiving part extending in the first direction, connecting respective distal ends of the pair of protruding parts together, and having the biasing member connected to the receiving part.

9. The pulse wave measuring device according to claim 8, wherein the receiving part is configured to limit a range of movement of the moving part by contacting the second arm.

10. The pulse wave measuring device recited according to any one of claims 1 to 9,
wherein the pulse wave measuring device is shaped substantially in a form of a clip, and
wherein the first arm and the second arm are positioned opposite each other across the wrist and curved to parallel at least a part of the wrist.

11. The pulse wave measuring device according to any one of claims 1 to 10, further comprising:
a first handle part connected to a proximal end of the first arm, extending opposite the first arm, and configured to swing about the first shaft; and
a second handle part connected to a proximal end of the second arm, extending opposite the second arm, and configured to swing about the first shaft.

12. The pulse wave measuring device according to any one of claims 1 to 11,
wherein the first arm includes a substrate mounting part on which a substrate connected to the pulse wave sensor is mounted, and
wherein a weight part with an adjustable center of gravity is formed in the second arm.

13. The pulse wave measuring device according to claim 12, wherein the weight part is formed at a distal end of the second arm.
